# EUROPÄISCHE PATENTSCHRIFT

(11) **EP 3 103 361 B1**
(45) Veröffentlichungstag und Bekanntmachung des Hinweises auf die Patenterteilung: **01.02.2023**
(21) Anmeldenummer: 15171382.3
(22) Anmeldetag: 10.06.2015
(51) Int. Cl.: A45C 5/00, A45C 11/00, A61B 5/103, A45C 13/02

(54) **VERFAHREN ZUR MESSUNG VON HAUTPARAMETERN FÜR DIE ANALYSE VON KOSMETISCHEN HAUTBEDÜRFNISSEN**
METHOD FOR MEASURING SKIN PARAMETERS FOR THE ANALYSIS OF COSMETIC SKIN NEEDS
PROCÉDÉ DE MESURE DE PARAMÈTRES PRINCIPAUX POUR L'ANALYSE DES BESOINS COSMÉTIQUES DE LA PEAU

(43) Veröffentlichungstag der Anmeldung: 14.12.2016
(73) Patentinhaber: Deters, Norbert, 45481 Mülheim (DE)
(72) Erfinder: Deters, Norbert, 45481 Mülheim (DE)

(56) Entgegenhaltungen:
- DE-A1- 4 032 959
- DE-T2-602005 004 349
- DE-T2-602005 004 349
- US-A- 3 316 993
- "Prospekt Hydrosensor / Skin Diagnostic SD 202", www.courage-khazaka.de, 10. April 2014 (2014-04-10), Seiten 1-1, XP055227937, Köln, Germany Gefunden im Internet: URL:http://www.courage-khazaka.de/index.ph p/de/alle-downloads/downloads-de/file/344- hydrosensor-skin-diagnostic-sd-202 [gefunden am 2015-11-12]

## Beschreibung

Die Erfindung betrifft ein Verfahren zur Messung von Hautparametern für die Analyse von kosmetischen Hautbedürfnissen gemäß dem Oberbegriff des Anspruchs 1.

Verfahren der eingangs genannten Art sind aus dem Stand der Technik, beispielsweise aus der DE 60 2005 004 349 T2, bekannt und dem Fachmann geläufig.

Für die Verfahren sind spezielle Messgeräte entwickelt worden, die zudem der Verkaufsförderung von Kosmetika und Behandlungen an den Verkaufspunkten Apotheke, Drogerie, Parfümerie, im Kosmetikinstitut oder in der dermatologischen Praxis dienen.

Die Geräte reichen dabei von kleinen, batteriebetriebenen Handgeräten bis zu Multifunktionskameras und Sondensystemen mit entsprechender Software, um die individuellen Hautbedürfnisse der Kunden zu ermitteln und die passende Pflege zu empfehlen.

Derweil werden insbesondere folgende Hautparameter an den Verkaufspunkten gemessen: Feuchtigkeit, Fett/Sebum, Pigmentierung und Hautrötung, Elastizität und biologisches Hautalter, pH-Wert der Haut, Hauttemperatur (Durchblutung der Haut), TEWL-Index (Qualität der Hautbarrierefunktion), Hautfarbe, Glanz auf der Haut, Fältchen und Falten, Poren, Flecken und Läsionen, Schuppigkeit/Desquamation, Talgdrüsenaktivität , Aktivität der Aknebakterien sowie Gesichtsfotografie mit verschieden Analysen.

Für die Messung des Hautfetts beispielsweise eignet sich die Photometrische Messung. Die photometrische Messung des Hautfetts ergänzt dabei idealerweise die Feuchtigkeitsmessung, um den individuellen Basishauttyp zu ermitteln. Hierzu wird ein Messgerät verwendet, bei dem ein spezielles Messband in einer Messkassette durchsichtig wird, wenn es mit dem Hautfett in Berührung kommt. Die Durchsichtigkeit wird im Gerät mit einer Photodiode gemessen und gibt den Hautfettgehalt an. Die Messzeit beträgt mehrere Sekunden. Eine Messkassette reicht für ca. 400 Messungen. Die Messung eignet sich insbesondere für Körperpflegeprodukten, Hautreinigungs- oder Anti-Akneprodukte.

Ein weiteres Messgerät ist eine Sonde, wobei die Sonde sowohl den Melaningehalt der Haut als auch die Rötung mittels Reflexion misst. Die hiermit verbundenen Pigmentierungsmessungen spielen bei der Empfehlung von Sonnenschutz und Aufhellungsprodukten eine Rolle. Die Rötung der Haut weist auf deren Sensitivität hin.

Die Hauttemperatur wird mit einem Messgerät zur Ermittlung der Infrarottemperatur ermittelt. Die Infrarottemperaturmessung der Haut lässt einen Rückschluss auf die Hautdurchblutung zu und stellt eine wichtige Messung im Bereich Cellulite oder durchblutungsfördernde Produkte dar.

Die Messung der viskoelastischen Eigenschaften der Haut stellt ein Indikator für das biologische Hautalter und ein ideales Promotionwerkzeug für elastizitätserhöhende Anti-Aging-Produkte dar. Es beruht auf der bekannten Ansaugmethode. Die Haut wird für einige Sekunden von Unterdruck aus dem Gerät angesaugt und dann mehrere Sekunden abgelassen. Hierbei werden die Fähigkeiten der Haut gemessen, sich dem Druck zu wiedersetzen und sich nach dem Wegfall des Druckes wieder in ihren Ausgangszustand zurückzubilden.

Die Bestimmung des Säureschutzmantels auf der Haut mittels eines pH - Messgeräts ist die Grundlage für die Empfehlung adäquater Hautpflege, Seifen und Reinigern.

Die Messung des Wassers, welches permanent in Form von Dampf durch die Haut entweicht, ist ein Indikator für Beurteilung der Hautbarriere. Die Messung findet in einer offenen Messkammer statt, in der je zwei Sensorenpaare (zur Messung von Feuchtigkeit und Temperatur) exakt den Wassergradienten der Haut ermitteln ohne dabei die Haut und ihr Mikroklima zu beeinflussen. Geringfügige Schädigungen der Barriere, die mit dem bloßen Auge nicht sichtbar sind, führen sofort zu einem erhöhten Wasserverlust. Hierfür stehen als Messgeräte ausgebildete Spezialsensoren zur Verfügung, die eine schnelle und genaue Messung erlauben.

Obwohl mittlerweile im Stand der Technik leistungsfähige Instrumentarien für die Messung der Hautparameter zur Verfügung stehen und nach wie vor eine große Nachfrage sowie ein großes Bedürfnis nach Kosmetika besteht, stehen dem Anbieter von Messungen von Hautparametern nur die bereits erwähnten herkömmlichen Verkaufspunkte Kosmetikinstitut oder in die dermatologischen Praxen zur Verfügung, um verkaufsfördernd tätig zu werden. Insbesondere ältere Menschen finden jedoch nicht ohne weiteres den für sie häufig mühsamen Gang zu den Verkaufspunkten. Auch lehrt die Erfahrung, dass manche Kunden in Verkaufspunkten, wie in Drogerien, Parfümerien oder aber auch in dermatologischen Praxen, die Messungen nur ungern durchführen lassen. Grund hierfür ist häufig, dass diese Kunden in Bezug auf Fragen ihrer eigenen äußeren Erscheinung nur sehr ungern einen öffentlichen Rahmen suchen, d.h. es liegen Beweggründe vor, die in der menschlichen Eitelkeit zu suchen sind. Hierdurch ergibt sich jedoch der Nachteil, dass der Verkauf von Kosmetika eine Einschränkung erfahren und nicht optimiert werden kann.

Es ist deshalb Aufgabe der vorliegenden Erfindung, diesen Nachteil zu vermeiden und den Verkauf von Kosmetika auf Basis der Messung von Hautbedürfnissen weiter zu steigern.

Diese Aufgabe wird mit den Merkmalen des Anspruchs 1 gelöst. Vorteilhafte Weiterbildungen der Erfindung ergeben sich aus den abhängigen Ansprüchen.

Die Erfindung sieht zunächst vor, dass die Untersuchung des Hauttyps an einem von einem Anbieter der Untersuchung und/oder von dem Verbraucher der Kosmetika frei bestimmbaren Standort durchgeführt wird, wobei das Messgerät zum frei bestimmbaren Standort mittels eines Transportmittels überführt wird.

Die grundlegende Idee der Erfindung ist es, die Untersuchung des Hauttyps des Verbrauchers unabhängig von den herkömmlichen Verkaufspunkten durchführen zu lassen, um den Verbraucher besser zu erreichen. Durch die Erfindung werden mobile Verkaufspunkte geschaffen, an denen vorab der Hauttyp eines Verbrauchers von Kosmetika untersucht wird.

Zur Umsetzung des erfindungsgemäßen Verfahrens wird als technisches Mittel ein Transportmittel eingesetzt, um die Messgeräte an den von dem Anbieter der Untersuchung bzw. von dem Verbraucher der Kosmetika bestimmbaren Standort zu bringen. Hierdurch wird die Anzahl der Verkaufspunkte erhöht. Im Gegensatz zu medizinischen Verfahren, die häufig große Gerätschaften erfordern, zeichnet sich das erfindungsgemäße Verfahren auch dadurch aus, das die Gegenstände für die Untersuchung, d.h. die aus dem Stand der Technik bekannten Messgeräte, in kompakter Form vorliegen und somit komplikationslos zu den gewünschten Standorten befördert werden können. Erstmalig erhält der Verbraucher von Kosmetika vorteilhafterweise durch die erhöhte Anzahl der Verkaufspunkte, die auch im privaten Umfeld liegen können, eine erhöhte Autonomie bei seiner Kaufentscheidung, was sich verkaufsfördernd auswirken sollte.

Das Messgerät wird erfindungsgemäß in einer Transportbox oder in einem Koffer als Transportmittel überführt. Da die Messgeräte in der Regel in kompakter Form vorliegen und kleine Abmessungen aufweisen, können die Messgeräte auch in kleinere Transportboxen integriert werden. Um den schonenden Transport zu gewährleisten, kann zudem zweckmäßigerweise vorgesehen sein, dass das Messgerät von Schaummaterial umgeben bzw. in dieses eingelegt wird.

Die Verwendung eines Koffers ist insbesondere geboten, wenn ein Handgriff für die Beförderung nützlich ist. Der Koffer kann vorzugsweise noch durch Ausstattungsmerkmale eines Kosmetikkoffers gekennzeichnet sein, sodass das Messgerät zusammen mit dem typischen Inhalt eines Kosmetikkoffers transportiert wird. Zusätzlich kann der Koffer bzw. der Kosmetikkoffer mit Kosmetikprodukten des Anbieters versehen werden.

Gemäß der Erfindung wird in das Transportmittel eine Computereinrichtung integriert. Die Computereinrichtung ist dabei mit dem Messgerät verbunden. Hierdurch werden erfindungsgemäß bei Vorhandensein einer entsprechenden Software die Ergebnisse der Hautuntersuchungen auch auf der Bildfläche eines Monitors dargestellt.

Um eine möglichst umfangreiche Beratung zu gewährleisten und den Hauttyp sicher zu ermitteln zu können, sieht die Erfindung auch vor, dass mehrere Messparameter während der Untersuchung verwendet werden.

Hierzu sieht eine vorteilhafte Weiterbildung der Erfindung vor, dass mehrere Messgeräte bei der Untersuchung eingesetzt werden. Beispielsweise können Messgeräte für die Messung der Feuchtigkeit der Haut, von Fett/Sebum, der Pigmentierung und der Hautrötung etc. zum Einsatz kommen.

Das Messgerät kann vorzugsweise mit einer externen Computereinrichtung verbunden werden. Der Anbieter der Untersuchungen kann so erst an dem gewählten Standort die Verbindung zwischen Messgerät und Computereinrichtung herstellen, was insbesondere bei größeren Computereinrichtungen geboten ist.

Um die Ergebnisse auch in Papierform vorliegen zu haben, ist es zweckmäßig, dass das Analysegerät mit einer Druckereinrichtung verbunden wird.

Eine weitere vorteilhafte Weiterbildung der Erfindung sieht vor, dass ein zusätzliches Transportmittel eingesetzt wird, wobei das zusätzliche Transportmittel motorisch angetrieben wird. Durch diese erfindungsgemäße Maßnahme kann ein Messgerät, das beispielsweise in einem Koffer transportiert wird, zusätzlich beispielsweise mittels eines Kraftfahrzeugs befördert werden. Hierdurch wird neben der schonenden Beförderung des Messgeräts, beispielsweise in einer Transportbox, auch die schnelle Beförderung, beispielsweise mittels eines Kraftfahrzeugs, gewährleistet.

Weitere Ziele, Vorteile, Merkmale und Anwendungsmöglichkeiten der vorliegenden Erfindung ergeben sich aus der nachfolgenden Beschreibung eines Ausführungsbeispiels anhand der Zeichnung.

Es zeigen in schematischer Darstellung:
- Fig. 1: in einem Blockdiagramm das Verfahren gemäß der Erfindung,
- Fig. 2: in einer perspektivischen Darstellung ein Transportmittel mit Inhalt des Verfahrens gemäß Figur 1 und
- Fig. 3: das Messgerät in dem Transportmittel aus Figur 2.

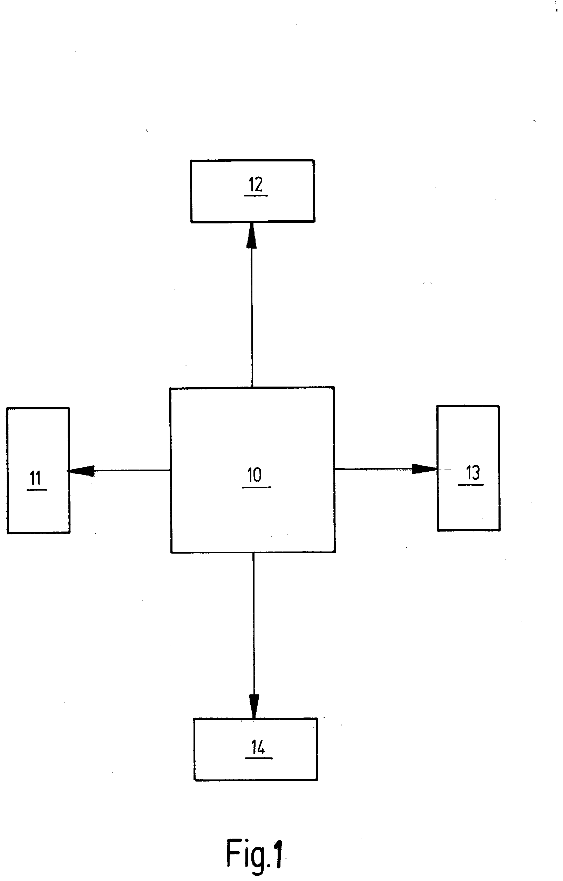

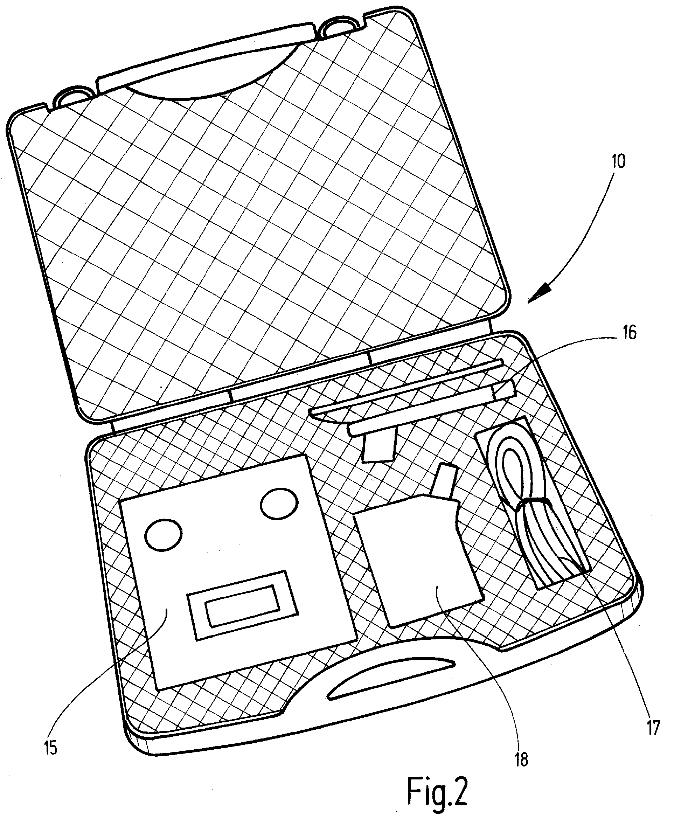

Figur 1 zeigt das erfindungsgemäße Verfahren zur Messung von Hautparametern für die Analyse von kosmetischen Hautbedürfnissen mittels der in Figur 2 gezeigten und in dem Transportmittel 10 befindlichen Messgeräte 15, 18, wobei im Rahmen der Messung und Analyse der Hauttyp eines Verbrauchers von Kosmetika untersucht wird.

Die Untersuchung des Hauttyps wird an von Verbrauchern der Kosmetika frei bestimmbaren Standorten 11, 12, 13, 14 durchgeführt. Bei den frei bestimmbaren Standorten 11, 12, 13, 14 handelt es sich ausschließlich um Privaträume der Verbraucher.

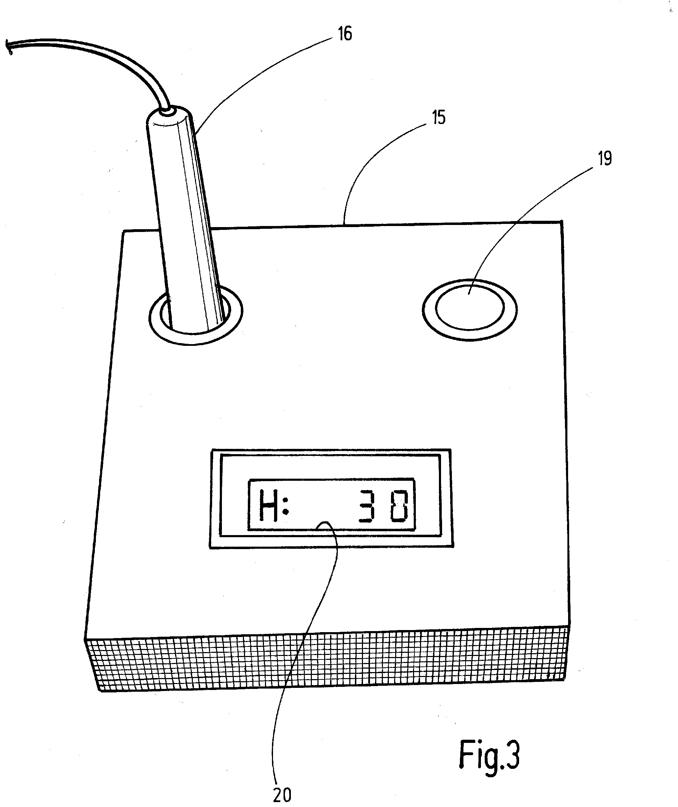

Um die Untersuchung der Hauttyps an den frei bestimmbaren Orten 11, 12, 13, 14 durchzuführen, werden in erfindungswesentlicher Weise die in Figur 2 gezeigten Messgeräte 15, 18 in dem Transportmittel 10 zum frei bestimmbaren Standort 11, 12, 13, 14 mittels des Transportmittels 10 überführt. Hierzu dient als Transportmittel 10 der in Figur 2 gezeigte Koffer. Zu dem Messgerät 15 gehört der Messstift 16, der mit dem Messgerät 15 verbunden werden kann und, wie Figur 3 verdeutlicht, in Kontaktaufnahmen 19 gesteckt wird, um den mit dem Stift 16 bzw. dem Messgerät 15 gemessenen Wert eines Hauptparameters mittels der Anzeige 20 des Messgeräts 15 darzustellen. In der in Figur 3 dargestellten Ausführungsform wird mit dem Messgerät 15 die Feuchtigkeit der Haut des Verbrauchers ermittelt. Hierzu wird die Spitze des Stifts 16 in einem unmittelbaren Kontakt mit der Haut des in den Figuren 1 bis 3 nicht gezeigten Verbrauchers gebracht.

Wie aus Figur 2 weiter hervorgeht, weist das Transportmittel 10 neben einem Ersatzkabel das Messgerät 18 auf, das sich für die Messung des Hautfetts des Verbrauchers eignet. Die Messung des Hautfetts erfolgt über eine photometrische Messung. Die photometrische Messung des Hautfetts ergänzt die Feuchtigkeitsmessung, um den individuelle Basishauttyp des Verbrauchers zu ermitteln. Hierzu wird ein spezielles Messband in einer Messkassette des Messgeräts 18 durchsichtig, wenn es mit dem Hautfett des Verbrauchers in Berührung kommt. Die Durchsichtigkeit wird in dem Messgerät 18 mit einer Photodiode gemessen und gibt den Hautfettgehalt an. Die Messzeit beträgt mehrere Sekunden.

Im Rahmen der Erfindung wird ein zusätzliches Transportmittel eingesetzt, wobei das zusätzliche Transportmittel motorisch angetrieben wird. Hierdurch werden die in dem Transportmittel 10 transportierten Messgeräte 15, 18 zudem mittels eines Kraftfahrzeugs zu den Standorten 11, 12, 13, 14 befördert. Das Transportmittel 10 wird dabei in dem Kraftfahrzeug verstaut.

## Patentansprüche

1. Verfahren zur Messung von Hautparametern für die Analyse von kosmetischen Hautbedürfnissen mittels mindestens eines Messgerätes (15, 18), bei dem der Hauttyp eines Verbrauchers von Kosmetika untersucht wird, wobei die Untersuchung des Hauttyps an einem von einem Anbieter der Untersuchung und/oder von dem Verbraucher der Kosmetika frei bestimmbaren Standort (11, 12, 13, 14) durchgeführt wird, wobei das Messgerät (15, 18) zum frei bestimmbaren Standort (11, 12, 13, 14) mittels eines Transportmittels (10) in Gestalt eines Koffers oder einer Transportbox überführt wird und wobei das Ergebnis der Hautuntersuchung visuell dargestellt wird, **dadurch gekennzeichnet, dass** in das Transportmittel (10) eine Computereinrichtung integriert wird und die Computereinrichtung mit dem Messgerät (15, 18) verbunden wird, wobei mittels einer Software die Ergebnisse der Hautuntersuchung auf der Bildfläche des Monitors dargestellt werden und mehrere Messparameter während der Untersuchung verwendet werden.

2. Verfahren nach Anspruch 1, **dadurch gekennzeichnet, dass** das Messgerät (15, 18) mit einer Druckereinrichtung verbunden wird.

3. Verfahren nach einem der vorhergehenden Ansprüche, **dadurch gekennzeichnet, dass** mehrere Messgeräte (15, 18) bei der Untersuchung eingesetzt werden.

4. Verfahren nach einem der vorhergehenden Ansprüche, **dadurch gekennzeichnet, dass** ein zusätzliches Transportmittel eingesetzt wird, wobei das zusätzliche Transportmittel motorisch angetrieben wird.

## Claims

1. A method for measuring skin parameters for the analysis of cosmetic skin requirements by means of at least one measuring device, in which the skin type of a consumer of cosmetics is examined, the examination of the skin type being carried out at a location which can be freely determined by a provider of the examination and/or by the consumer of the cosmetics, wherein the measuring device is transferred to the freely determinable location by means of a transport means in the form of a suitcase or a transport box and wherein the result of the skin examination is visually displayed, **characterized in that** a computer device is integrated into the transport means and the computer device is connected to the measuring device, wherein by means of software the results of the skin examination are displayed on the screen of a monitor and several measuring parameters are used during the examination.

2. The method according to claim 1, **characterized in that** the measuring device is connected to a printer device.

3. The method according to any one of the preceding claims, **characterized in that** several measuring devices are used during the examination.

4. The method according to any one of the preceding claims, **characterized in that** an additional transport means is used, said additional transport means being motor-driven.

## Revendications

1. Procédé de mesure de paramètres cutanés pour l'analyse des besoins cosmétiques de la peau au moyen d'au moins un appareil de mesure, dans lequel le type de peau d'un consommateur de produits cosmétiques est examiné, l'examen du type de peau étant effectué à un endroit pouvant être déterminé librement par un fournisseur de l'examen et/ou par le consommateur des produits cosmétiques, l'appareil de mesure étant transféré à l'emplacement librement déterminable à l'aide d'un moyen de transport sous la forme d'une valise ou d'une boîte de transport et le résultat de l'examen de la peau étant représenté visuellement, **caractérisé en ce qu'**un dispositif informatique est intégré dans le moyen de transport et le dispositif informatique est relié à l'appareil de mesure, les résultats de l'examen de la peau étant représentés sur la surface d'image d'un moniteur au moyen d'un logiciel et plusieurs paramètres de mesure étant utilisés pendant l'examen.

2. Procédé selon la revendication 1, **caractérisé en ce que** l'appareil de mesure est connecté à un dispositif d'impression.

3. Procédé selon l'une des revendications précédentes, **caractérisé en ce que** plusieurs appareils de mesure sont utilisés pendant l'examen.

4. Procédé selon l'une des revendications précédentes, **caractérisé en ce qu'**un moyen de transport supplémentaire est utilisé, où le moyen de transport supplémentaire étant entraîné par un moteur.
